(19) **Europäisches Patentamt / European Patent Office / Office européen des brevets**

(11) **EP 1 945 125 B1**

(12) **EUROPEAN PATENT SPECIFICATION**

(45) Date of publication and mention
of the grant of the patent:
**03.04.2013  Bulletin 2013/14**

(21) Application number: **06839762.9**

(22) Date of filing: **05.11.2006**

(51) Int Cl.:
***A61B 19/00*** *(2006.01)*    ***A61B 17/14*** *(2006.01)*

(86) International application number:
**PCT/US2006/060649**

(87) International publication number:
**WO 2007/056743 (18.05.2007 Gazette 2007/20)**

(54) **SURGICAL SAW BLADE WITH FEATURES RECOGNIZABLE BY A SURGICAL NAVIGATION SYSTEM FOR DETERMINING THE CHARACTERISTICS OF THE SAW BLADE AND METHOD OF USING SAME**

CHIRURGISCHES SÄGEBLATT MIT MERKMALEN DIE DURCH EIN CHIRURGISCHES NAVIGATIONSSYSTEM ERKENNBAR SIND UM DIE SÄGEBLATTMERKMALEN ZU BESTIMMEN UND VERWENDUNGSVERFAHREN DAFÜR

LAME DE SCIE CHIRURGICALE AVEC CARACTÉRISTIQUES RECONNAISSABLES PAR UN SYSTÈME DE NAVIGATION CHIRURGICALE POUR DÉTERMINER LES CARACTÉRISTIQUES DE LA LAME DE SCIE ET SON PROCÉDÉ D'UTILISATION

(84) Designated Contracting States:
**DE FR GB IT**

(30) Priority:  **09.11.2005   US 735063 P**

(43) Date of publication of application:
**23.07.2008   Bulletin 2008/30**

(73) Proprietor: **STRYKER CORPORATION
Kalamazoo, MI 49002 (US)**

(72) Inventors:
• **MALACKOWSKI, Don
Schoolcraft, MI 49087 (US)**

• **CULP, Jerry
Kalamazoo, MI 49001-6197 (US)**
• **MOCTEZUMA DE LA BARRERA, José Luis
79104 Freiburg (DE)**

(74) Representative: **Röthinger, Rainer
Wuesthoff & Wuesthoff
Patent- und Rechtsanwälte
Schweigerstrasse 2
81541 München (DE)**

(56) References cited:
**EP-A- 1 444 962       WO-A-2005/063139
DE-A1- 10 239 710     US-A1- 2005 154 296**

## Description

Field of the Invention

This invention relates generally to saw blades and other planar shaped accessories used to perform surgical procedures. More particularly, this invention relates to a system and method for precisely locating such cutting accessories with a surgical navigation system.

Background Of The Invention

A surgical navigation unit is sometimes used to aid the performance of a surgical procedure. Generally, a surgical navigation unit comprises one or more trackers, a localizer and a processor. The trackers are attached to the instruments and surgical tools employed to perform the procedure. The localizer receives signals from the trackers. Based on these signals, the localizer generates signals representative of the position and orientation of the trackers. The processor receives the signals generated by the localizer.

Preloaded into the processor is an electronic map of the site on or in the body of the patient at which the surgical procedure is being performed. The processor, based on signals received from the localizer, determines the position and orientation of the trackers. By extension, the processor also determines the positions and orientations of the surgical instruments and tools to which the trackers are attached. Based on these data, the processor determines the position and orientation of the surgical instruments and tools relative to the surgical site within the patient. The processor generates an image, presented on a display, which indicates the location of the surgical instrument or tool relative to the surgical site. A surgical navigation system thus functions as means to ensure a surgical instrument or tool is properly positioned relative to the surgical site.

Sometimes the surgical tool or instrument to which the tracker is attached is not the actual device physically applied to the surgical site. Instead, a device, sometimes referred to as a "cutting accessory," is applied to the surgical site. For example, one surgical instrument (tool) employed to perform a surgical procedure is the powered handpiece. Internal to the handpiece is motor with a rotating shaft. Various types of cutting accessories such as drill bits or reamers are removably coupled to the handpiece shaft.

After a cutting accessory is attached to this type of handpiece, the distal end of the accessory, the tip of the drill or the forward end of the reamer head, is touched to a calibration unit. Prior to this step, data are stored in the processor containing the location of the calibration unit. The processor thus contains data from the tracker integral with the handpiece that indicates the position of the handpiece and data indicating the position of the calibration unit. By extension, from the touching of the distal end of the cutting accessory to the calibration unit, the processor determines the location of the distal end of the cutting accessory relative to the handpiece. Based on these data, the surgical navigation unit generates data that indicates the position of the cutting accessory as the handpiece to which the accessory is attached is moved. These data are used to generate data indicating the position of the distal end of the cutting accessory relative to the surgical site.

The above system works well for a cutting accessory that, on attachment to a handpiece, remains at a fixed location. Such accessories include rotating devices such as reamers or drills.

However, some cutting accessories shift position relative to the surgical tools to which they are attached. One such cutting accessory is the saw blade. Generally, a saw blade moves in an oscillating or reciprocating pattern. This enables the teeth, at the head of the blade, to cut through the tissue against which the blade is applied. Given that the blade head moves relative to the handpiece to which it is attached, it has proven difficult to simply touch the blade head to a calibration unit in order for the navigation system processor to generate data that indicates position of the blade head relative to the handpiece.

Furthermore, some handpieces include indexing mechanisms. The indexing mechanism shifts the angular orientation of the cutting accessory (saw blade) relative to an axis of the handpiece. This arrangement facilitates the positioning of the cutting accessory relative to the handpiece so it is in a position that allows the surgeon to perform the procedure with no or minimal ergonomic strain. Again, for a surgical navigation system to be able to determine the position of the blade head, it is necessary for the system to be provided with data indicating the indexed position of the blade.

A position sensor could be attached to the subassembly integral with the handpiece to which the blade is attached. Often, this subassembly is a blade mount. The sensor would generate a variable signal as a function of both the indexed position of the mount and its position in its reciprocal path of travel. Based on these sensor signals, the surgical navigation system processor could generate data that indicates the position of the blade head. However it is difficult to provide this type of sensor assembly. This difficult arises, in part, because this sensor assembly, like the other components of the handpiece, must be designed to withstand the rigors of autoclave sterilization (placed in a water vapor saturated environment at 132°C at 2,1 bar (270°F at 30 psi) (Gage)).

DE 102 39 710 discloses a handpiece having a tracker thereon which can be tracked by a surgical navigation system. A transponder is located on a proximal end of each of a plurality of surgical workpieces for the handpiece, whereby the surgical navigation system comprises a reader for reading an individualized identifier from the transponder of a particular workpiece to obtain corresponding geometric data for the workpiece, and uses this data to determine a location of the workpiece relative to a surgical site. The two part from of claim 1 and 2 is based on this document.

US 2005/0154296 discloses a surgical navigation system, wherein shafts of surgical workpieces which can be inserted into a surgical handpiece have a single indentation which is placed in a specific location for each surgical workpiece. Applying a pointer to the indentation and determining a distance between the indentation and a tracker placed on the surgical handpiece, the type of workpiece is determined. For a rotationally asymmetric workpiece, a surgeon can select on a screen of the surgical navigation system an orientation of the surgical workpiece corresponding to the orientation when being calibrated.

Thus, to date, it has proven difficult to provide a means for providing data to a surgical navigation system that indicates the position and orientation of the head of a cutting accessory, such as a saw blade, that engages in translational motion.

Summary of the Invention

This invention relates to exchangable surgical cutting accessories, such as saw blades. The cutting accessory of this invention is designed for integration into a surgical navigation system so that the navigation system generates data precisely indicating the position of the distal end of the accessory, the end applied to the surgical site.

A surgical saw blade, a surgical tool system, and a method for determining a position of teeth of a surgical saw blade according to the independent claims are provided. Preferred embodiments are disclosed in the dependent claims.

The cutting accessory of this disclosure may be formed with one or more reference features. The reference features are located at precisely known positions on the cutting accessory. For example, in a version of the invention when the cutting accessory is a saw blade, the features are located at known positions relative to the blade teeth.

The cutting accessory of this disclosure may be used by attaching the accessory to the complementary surgical tool, handpiece, used to actuate the accessory. A pointer is placed on the reference features. Based on the position data generated by the pointer, a processor integral with the surgical navigation system derives data that indicates the position and orientation of the distal end of the cutting accessory relative to the complementary handpiece. Based on these data, the processor generates data indicating the location of the distal end of the cutting accessory relative to the surgical site.

It is still another aspect of this invention to provide the cutting accessory with reference features that are in a specific pattern unique to the specific type of the accessory. As a consequence of the pointer identifying these features, the surgical navigation system processor extracts data describing the specific patterns. Once the pattern data are determined, the system of this invention, by reference to look-up data, identifies the type of cutting accessory. Once this identification process is performed, the processor retrieves data used to regulate the operation of the handpiece so that handpiece operates in a manner based suited for actuating the cutting accessory. These data are then used to regulate handpiece operation.

Brief Description of the Drawings

The invention is pointed out with particularity claims. The above and further features of the invention are better understood from the following Detailed Description taken in conjunction with the accompanying drawings in which:

Figure 1 depicts a surgical navigation system into which the cutting accessory identification system of this invention is incorporated;

Figure 2 is perspective view of how a cutting accessory of this invention is attached to a surgical tool such as a powered handpiece;

Figure 3 is a plan view of one cutting accessory, more particularly a saw blade, constructed in accordance with this invention;

Figures 4A and 4B are a flow chart of the process steps executed to determine the type of cutting accessory attached to the tool and how the distal end location of the cutting accessory is determined according to this invention;

Figure 5 illustrates how a pointer is touched to a saw blade reference point according to this invention;

Figure 6 is a diagrammatic illustration of the curve defined by the reference divots of the blade of Figure 3;

Figure 7 depicts the types of data stored in a blade definition file integrally associated with a particular blade of the invention;

Figure 8 is a reproduction of image of a surgical navigation system of this invention indicating the location of the cutting accessory;

Figure 9 is a flow chart of an alternative process of this invention how the system of this invention recognizes that a particular type of accessory has been fitted to a surgical tool;

Figure 10 is a plan view of a second saw blade, constructed in accordance with this invention;

Figure 11 is a flow chart of an additional alternative process of this invention to recognize the type of accessory fitted to a tool;

Figure 12 is partial depiction of an alternative blade definition file;

Figure 13 is a plan view of a third saw blade constructed in accordance with this invention;

Figure 14 is a flow chart of an additional alternative process of this invention to recognize the type of accessory fitted to a tool; and

Figure 15 is a diagrammatic illustration of the curve reconstructed by reference to the divots of the blade of Figure 13.

Detailed Description

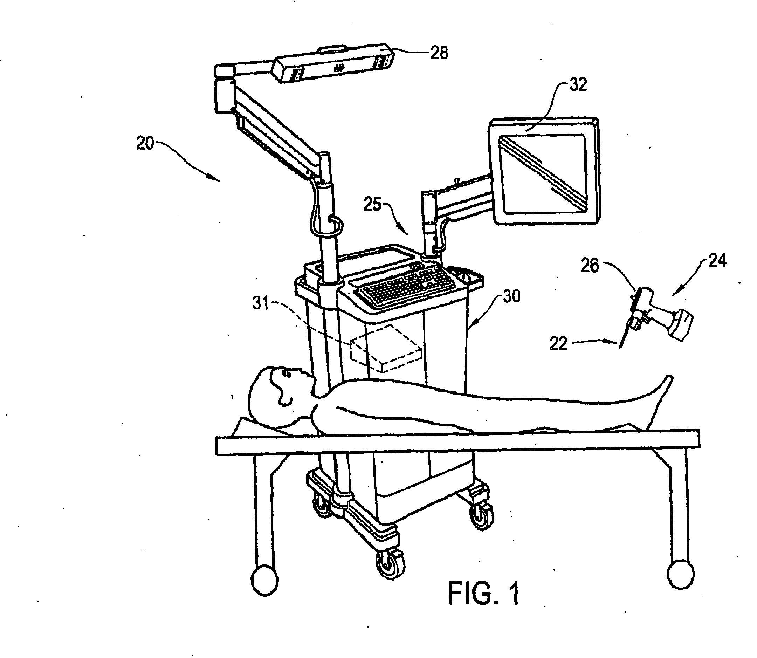

A surgical system 20 of this invention, including a cutting accessory 22, is shown in Figure 1. System 20 includes a tool 24 used to position accessory 22 at a surgical site and actuate the accessory. In the described version of the invention, cutting accessory 22 is a saw blade and tool 24 is a handpiece that actuates the blade. The system 20 also includes a surgical navigation system 25. Surgical navigation system 25 includes a tracker 26 mounted to the tool 24. There is also a localizer 28.

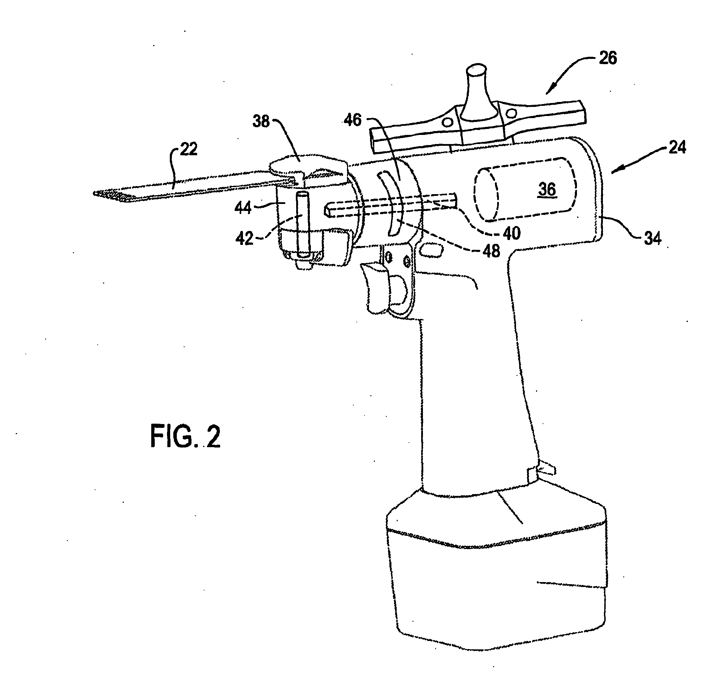

Tracker 26 actively or passively broadcasts a specific form of energy from one or more emitters or reflectors (resonators) (Figure 2). Some trackers 26, for example, emit infra-red light or visible light. Other trackers emit RF or electromagnetic energy. Still other trackers emit ultrasonic energy. Localizer 28 monitors the position of the tracker 26. Specifically, the localizer 28 contains one or more receivers (not illustrated) capable of monitoring the energy emitted by the tracker 26. The receivers generate specific signals as a function of the direction from which the energy is transmitted. A processor 30, also part of surgical navigation system 25, receives the signals generated by the localizer receiver. Processor 30, based on the localizer receiver signals, generates signals representative of the position and orientation of the tracker 26 in free space.

A more detailed explanation of how a surgical navigation system operates is contained in the Applicant's U.S. Patent Application No. 10/677, 874, *SURGERY SYSTEM,* filed October 2, 2003, U.S. Patent Publication No. US 2004/0073279 Al. It should be appreciated that the system and method of this invention is not limited to versions of the invention using the above incorporated-by-reference navigation system. Other navigation systems may be incorporated into this invention. This includes navigations systems designed so that, based on signals transmitted by the localizer, components internal to the trackers generate signals representative of tracker position and orientation.

Tracker 26 is firmly attached to the tool 24. The geometry and dimensions of the tool 24 are known. Therefore, by extension, from the data indicating the position and orientation of the tracker 26, processor 30 generates data indicating the position and orientation of the tool 24. As discussed below, in this invention, processor 30 is also provided with data indicating the position and orientation of the cutting accessory 22 relative to the tool 24. Based on these data, data indicating the position and orientation of the cutting accessory 22 as it is moved are generated.

Prior to the start of the surgical procedure, data defining the location of the site on or within the patient at which the surgical procedure is performed are loaded into the processor 30. More particularly, these data are stored in a memory integral with the processor 30 represented by phantom block 31. Based on these data and the processor-generated data indicating the position of the cutting accessory 22, processor 30 generates image-defining data that indicates the location of the cutting accessory 22 relative to the surgical site. These image defining data are used to present on a display 32 an image that indicates the location at the surgical site at which the cutting accessory 22 is located.

Simultaneously with the monitoring of the position of the tracker 26, localizer monitors the energy emitted by one or more marker, sometimes called fiducials 33 (one shown) affixed to the patient. Fiducials 33 include components that, like the members integral with the tracker 26, emit energy that can be sensed by localizer 28. The positions of the fiducials are tracked. Based on the data representative of fiducial position, processor 30 generates what is considered to be a dynamic reference frame for the body of the patient. The reference frame is considered "dynamic" because during the procedure, there may be some movement of the patient. Then, during the procedure, processor 30 maps this previously generated image-defining data of the patient's tissue onto the dynamic reference frame.

Figure 2 illustrates a handpiece 24 to which a cutting accessory 22 and a tracker 26 are attached. Handpiece 24 includes a housing 34 in which a motor, represented by phantom cylinder 36, is mounted. Blade 22 is fitted in a blade mount 38 located distally forward of handpiece housing 34. ("Distal", it shall be understood, means toward the surgical site to which the blade 22 and handpiece 24 are directed. "Proximal", means away from the surgical site.) A drive mechanism, represented by a phantom bar 40, converts the rotary motion generated by the motor 34 into motion that reciprocates the blade mount 38 and, therefore, blade 22 in a back and forth pattern. A coupling assembly, represented in phantom by cylindrical rod 42, releasably holds the proximal end of the saw blade 22 to the blade mount 38.

Blade mount 38 is rotatably fitted in a head 44 located forward of handpiece housing 34. The head 44 is rotatably fitted in a collar 46 that extends forward from and is integral with the handpiece housing. A indexing mechanism, represented by phantom ring 48, releasably holds the handpiece head 44 in a fixed angular orientation around the longitudinal axis of the collar 46. This arrangement allows the angular orientation of the blade 22 to be set to an ergonomically convenient location in order to perform the intended surgical procedure.

The Applicant's Assignee's U.S. Patent Application No. 60/699,315, *SURGICAL SAGITTAL SAW WITH QUICK RELEASE INDEXING HEAD AND LOW BLADE-SLAP COUPLING ASSEMBLY,* filed 14 July 2005, discloses a coupling assembly for holding the blade to the blade mount and an indexing assembly for selectively positioning the head. Both assemblies are understood to be examples of the types of assemblies that can be incorporated into the system 20 of the invention. This invention is not limited to systems including one or both of these assemblies.

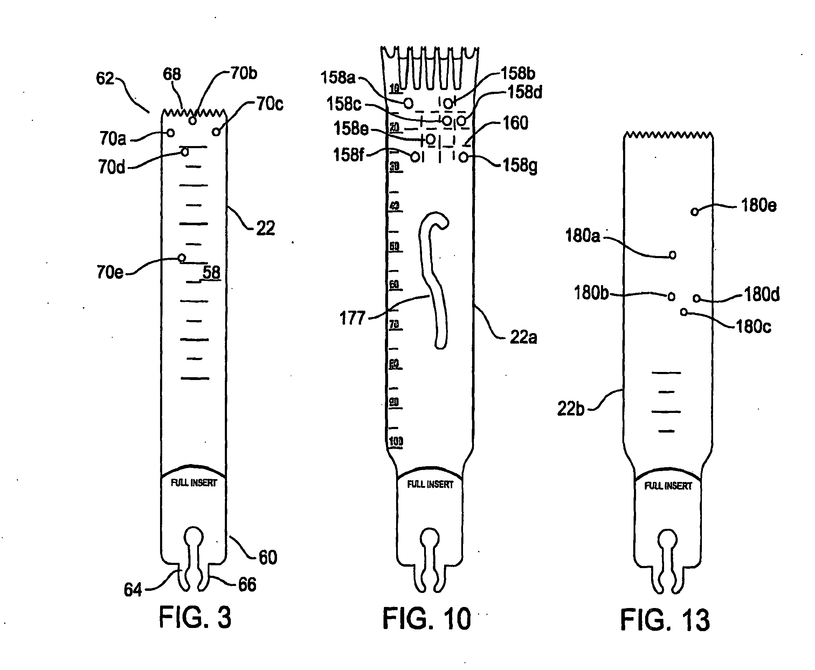

Blade 22, seen best in Figure 3, includes opposed proximal and distal ends 60 and 62, respectively. Blade proximal end 60 is formed with a coupling features 64, here represented by proximally extending tines 66. Coupling features 64 engage the coupling assembly integral with the handpiece 24. It should be appreciated that this invention is independent of the coupling features of the blade in which the invention is incorporated. The blade distal end 62 is formed with cutting teeth 68. The geometry of the blade teeth is not relevant to this invention. As discussed below, this invention provides data used to determine the geometry of blade teeth.

Saw blade 22 is further formed with reference features. In the blade of Figure 3, these reference features are three divots 70a, 70b and 70c. Divots 70a-70c are formed on the blade so as to be a precisely known distance from teeth 68.

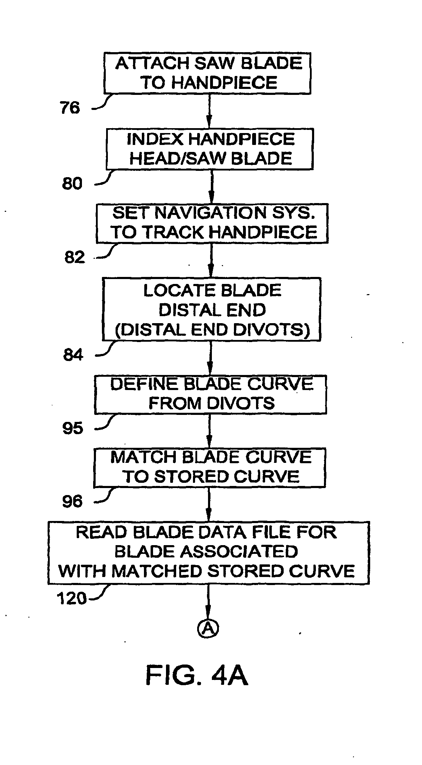

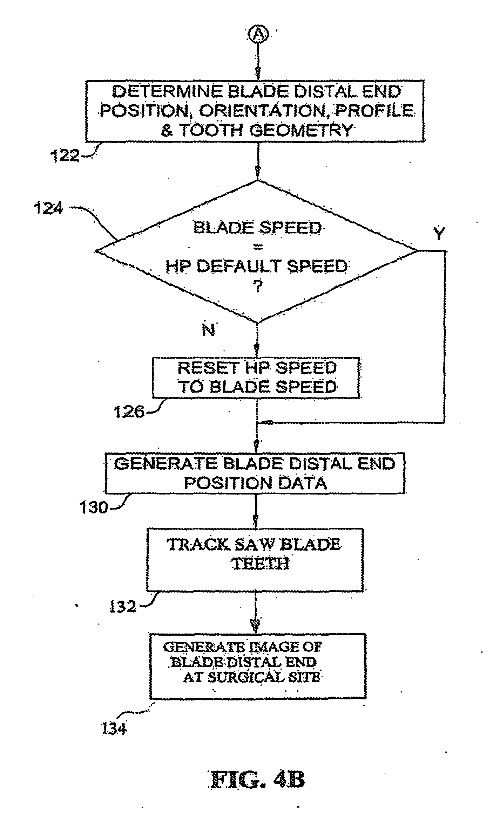

The handpiece with blade assembly of this invention for operation by first attaching the saw blade 22 to the handpiece 24, step 76 of Figure 4A. In step 80, the handpiece indexing mechanism is set to place the blade mount and saw blade in the desired position to accomplish the surgical process. Also during the initial set up process, the appropriate steps are then executed to cause the surgical navigation system to track the tracker 26 and so that its processor 30 generates data indicating the position and orientation of the handpiece 24. In Figure 4A this is represented by step 82. This step is shown as being executed after the blade is loaded and the indexing mechanism is set. This step may be executed earlier in the process.

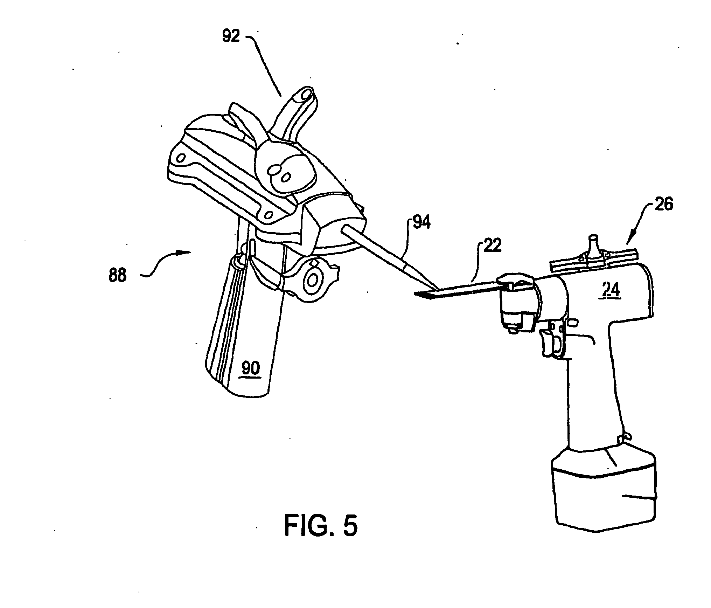

Once the surgical navigation system30 starts tracking the handpiece 24, a step 84 is executed to locate the blade distal end 62. As seen by reference to Figure 5, in step 84, a pointer 88 is touched to each of the divots 70a, 70b and 70c. Pointer 88 is a tool that is part of the surgical navigation system 25. The pointer 88 includes a handle 90 to which a tracker 92 is attached. (In the Figures, trackers 26 and 92 are aesthetically different.) An elongated tip 94 extends forward from the handle 90. The pointed end of the tip is the pointer components touched to the divots 70a-70c.

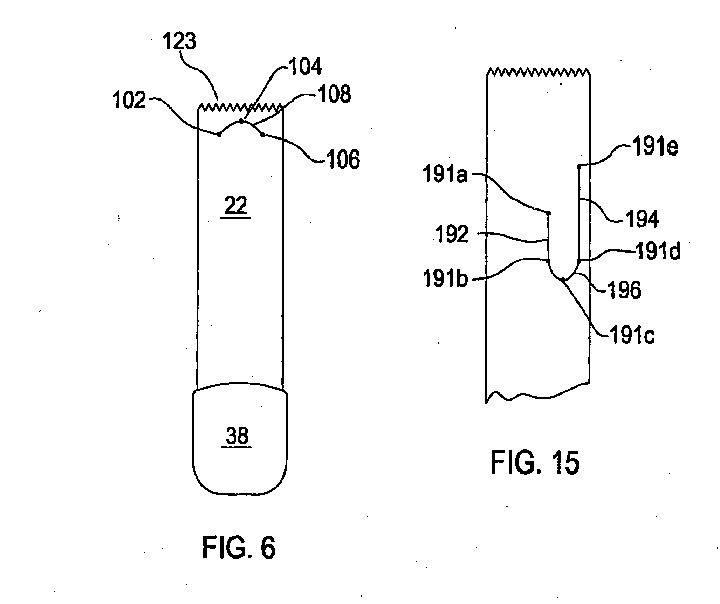

As the pointer tip 94 is touched to each recess 70a, 70b and 70c, localizer 28 monitors the signals emitted by the pointer tracker 92. Based on the signals emitted by the localizer, the surgical navigation unit processor 30 determines the position of each recess 70a-70c. In a step 95, processor 30 uses a curve construction algorithm to determine the shape and position of a curve defined by divots 70a-c. Examples of such curve construction algorithms that can be employed are curve fitting regression algorithms, polynomial curve fitting algorithms or curve fitting regression algorithms. Some curve construction algorithms require the knowledge of the three or more points (divots). However, some curve construction algorithms only require the knowledge of the location of two points (divots). For example, if the curve construction algorithm is based on there being an arc of predefined curvature, section of a circle, between the points, only the location of two divots are required. In still other versions of the invention, the "curve" is a set of line segments that form a spline. Again, these algorithms should be understood to be exemplary, not limiting. In Figure 6, points 102, 104 and 106 represent the locations of divots 70a, 70b and 70c, respectively. Curve 108 is the curve generated in step 95 that is fit to points 102, 104, and 106.

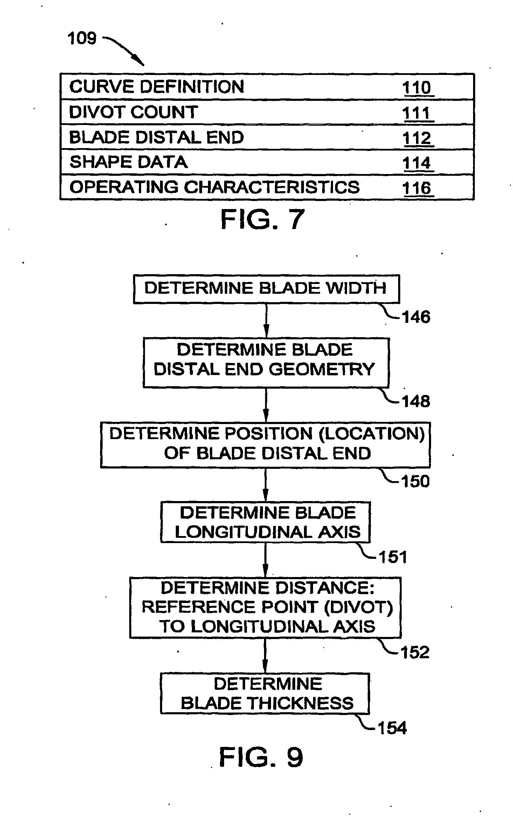

Once the curve 108 is defined, in a step 96, the processor determines to which one of a plurality of stored blade distal end curve geometries the curve defined in step 95 corresponds. These blade curve geometries are stored in the processor memory 31. Each stored blade curve geometry is associated with a blade definition file in the memory that is unique to a specific type of blade. Each file, one represented by block 109 of Figure 7, contains a curve definition data field 110 with data that mathematically defines the curve integral with the blade 22. Curve definition field 110 includes a secondary divot count field 111. Divot count field 111 contains data indicating the number of divots that are used to define the curve.

Each blade definition file 109 also contains a data field 112 contains data indicating the distance from the defined curve to the actual teeth blade. A shape data field 114 contains data that define the actual geometry of the blade distal end 62. This geometry includes both the shape, perimeter profile of the blade distal end and the geometry of the teeth. Shape data field 114 also contains data indicating the thickness of the blade. An operating characteristics data field 116 contains data that define the operating characteristics of the blade. These characteristics include preferred, (default) blade speed and the maximum speed at which the blade should be actuated.

In step 96, a shape matching algorithm is used to match the curve 108 generated in step 95 to one of the stored curves from one of the fields 110. Suitable shape matching algorithms that can be employed are based on the radial distance from the center of mass (shape histogram), isotropic scaling or anisotropic scaling. This list should be understood as exemplary, not limiting. Additional matching of the blade to one of the blade definition files 109 is performed by matching the number of divots pointed to on the blade to define the curve with the number of divots in the divot count fields 111. Once the blade is matched with one of the blade definition files 109, in step 120, the rest of the data in the blade definition file 109 are read.

In a step 122, based on the position and orientation of the curve 108 and the data read from the selected blade definition file 109, processor 30 determines the position, shape and orientation of the blade distal end 62. Specifically, based on the data in the blade distal end data field 112, processor 30 determines how far forward of curve 108 the blade distal end is located. Based on the data in the shape data field 114, the processor determines that the shape of the blade distal end and the tooth geometry. In Figure 6 this is represented by the saw tooth plot 123 located forward of curve 108. Thus, this particular blade has a slightly curved distal end with saw tooth teeth.

In a step 124, the system of this invention also determines if the operating speed of the handpiece can be reset. Generally a handpiece is set so that the motor causes the blade mount to oscillate at a rate equal to the slowest oscillation rate of any of the potential blades that may be fitted to the handpiece 24. Typically, this rate is between 10,000 and 15,000 cycles per minute. (A "cycle" is one complete back and forth oscillation of the blade mount 38.) Some blades can be operated at higher cycle rates. Thus, in step 124 the processor 30 determines if the data field 116 for the blade indicates the blade can be operated at a rate greater than the low cycle rate default rate and what the default rate is for that blade.

If, in step 124 it is determined the blade can be operated at a speed greater than the default low cycle rate setting, processor 30 resets the operating speed of the motor, step 126. The Applicants' Assignees' U.S. Patent Application No. 60/694,592, filed 28 June 2005, *Powered Surgical Tool With Sealed Control Module,* discloses one such assembly for resetting the operating speed of the motor. Alternatively, if in step 124 the data from field 116 indicates the cycle rate is the lowest cycle rate, step 126 is bypassed.

As a consequence of the execution of steps 82 and 122, processor 30 contains data indicating the position of the handpiece 24 and the location of the blade distal end 62 (saw teeth). Based on these data, processor 30, in step 130, generates data indicating the position and distance from the handpiece 20 to the teeth 68. Specifically, in step 130, the processor 30 generates data indicating the index orientation of the blade 22. Processor 30, in step 130, also generates data that indicates the position of the blade distal end 62 relative to a fixed reference of the blade mount. Diagrammatically in Figure 6, this is seen by the positioning of tooth plot 123 a specific distance forward of the blade mount 38.

Once step 130 is performed, system 20 of this invention is ready for use. As a consequence of the tracking of handpiece 24 and the known location of the blade distal end relative to the handpiece, the position and orientation of the distal end teeth 68 of the saw blade are known. Mathematically, this relationship is expressed as follows:

$$\vec{x}_{L \to ST} = \vec{x}_{L \to H} + R_{L \to H} \cdot \vec{x}_{H \to ST} \qquad (1)$$

$$R_{L \to ST} = R_{L \to H} \cdot R_{H \to ST} \qquad (2)$$

Here, $\vec{x}_{L \to ST}$ is the vector from the localizer to the saw blade teeth. Vector $\vec{x}_{L \to H}$ is the vector from the localizer to the handpiece. Vector $\vec{x}_{L \to ST}$ is the vector from the handpiece to the saw blade teeth. Matrix $R_{L \to ST}$ is the rotational matrix from the localizer to the saw blade teeth. Matrix $R_{L \to H}$ is the rotational matrix from the localizer to the handpiece. Rotational matrix $R_{H \to ST}$ is the matrix from the handpiece to the saw blade teeth. Thus, as a consequence of the system 20 being able to track the handpiece, the system is able to inferentially track the position of the distal end of the saw blade, step 132.

Because the saw blade oscillates back and forth at a relatively high speed, appx 10,000 to 20,000 cycles per minute, the processor does not generate data indicating the instantaneous position of the blade 22. Instead, in step 134, processor 30 generates an image of the blade 106 that represents the whole of the path of movement of the blade.

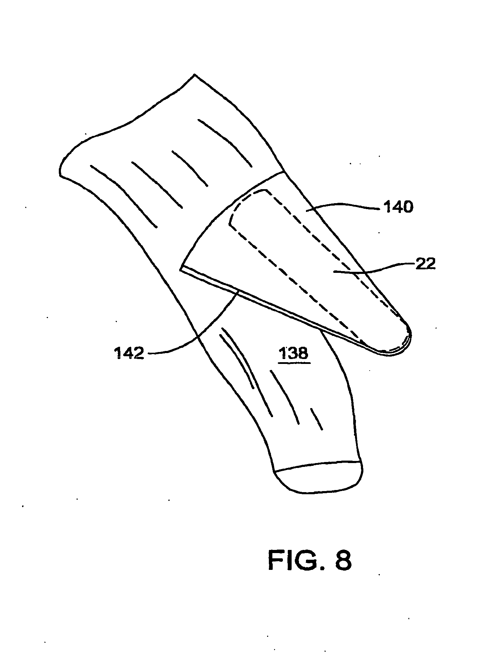

Consequently, as represented by Figure 8, surgical personal viewing the surgical site on display 32 see the whole of the space subtended by the blade as a consequence of its actuation as subtending the adjacent tissue. In this Figure, a bone 138, such as femur, is shown as being the body tissue on which the surgical procedure is being performed. Arcuate slice 140 is the image presented on the display to illustrate the space within which the blade is oscillating. Within slice 140, shown for reference only and not part of the generated image, blade 22 is depicted as being a phantom element within slice 140.

It should further be appreciated that, as part of the generation of the image, in step 134, processor 30 generates data that represents the thickness of the blade 22 and the space subtended by the blade. These data are based on the data retrieved from the shape data field 114. In Figure 8, this is represented by the lateral side wall 142 of slice 140. The generated image thus provides surgical personal a means to readily determine the extent to which the blade teeth are being applied against the tissue to which they should be applied and if the blade is encroaching tissue against which it should not be applied.

Thus, the system of this invention provides surgical personnel with a means to view at a surgical site with a surgical navigation system a saw blade or like cutting accessory that shifts position relative to the handpiece to which it is attached.

The system and method of this invention may employ other means to, from the divot positions, derive data defining the distal end geometry of the saw blade.

In one such alternative method. The curve defining divots 70a-c are located at a precise distances from edge surfaces that define the blade distal end 62. Specifically, divots 70a and 70c, the divots that define the opposed ends of the curve, are each located defined distances from the side edges of the blade distal end. Each of the divots, 70a, 70b and 70c, are located a set distance proximally rearward from most distal tips of the blade teeth 68. Each of these distances is constant for all blades 22 used in the system, regardless of blade length, width or shape

In this structure of the invention, there are fourth and fifth divot 70d and 70e, respectively. Divot 70d is located rearwardly of divots 70a-70c. Divot 70d is further formed on the face of the saw blade 22 to be located a defined distance from the line defined by divots 70a and 70c. Divot 70e is formed on the blade so as to be offset a specific distance from the longitudinal axis that extends through the blade.

The process steps executed to determine blade geometry in this embodiment of the invention are now described by reference to the flow chart of Figure 9. More particularly, the flow chart of Figure 9 illustrates the steps performed after step 84 is executed. In this version of the invention, during the execution of step 84, pointer 88 is touched to all four divots 70a-70d to determine their individual positions.

Once the positions of divots 70a-70d are determined, in a step 146 the width of the blade is determined. This width is determined by first determining the distance between the two opposed outermost divots. In the present example, these are divots 70a and 70c. Once this distance is determined, processor 30 determines the width of the blade according to the following formula:

$$\texttt{Blade Width = Distance Between Outermost}$$
$$\texttt{Curve Defining Divots + K} \qquad (3)$$

Here, K is constant stored in the processor memory 31. Constant K is the same for all blades.

Distal end blade geometry is then determined in a step 148. In step 148 curve fitting algorithm that may be one of algorithms employed in step 95 is used to establish the curve defined by divots 70a, 70b and 70c. The curve generated in step 148, which may be a line, is the geometry of the distal end perimeter of the blade 22.

In a step 150, processor 30 determines the position of the blade distal end 62. This determination is made according to the following formula:

$$\texttt{Blade Distal End Position =}$$
$$\texttt{Curve Position + W} \qquad (4)$$

Here, W is a constant also stored in processor memory 31. Constant W is identical for all blades.

Alternatively, in step 150, processor 30 determines the distance D between divot 70b and the line defined by divots 70a and 70c. Then in step 144, the blade distal end position is determined by one of the following formulas:

$$\texttt{Position of the Line Defined}$$
$$\texttt{By Divots 70a and 70c}$$
$$\texttt{+ Distance Between The Line and Divot 70b}$$

$$\texttt{+ W (Constant)}$$
$$\texttt{= Blade Distal End Position} \qquad (4a)$$

or

$$\text{Position of the Line Defined}$$
$$\text{By Divots 70a and 70c}$$
$$+ \quad \text{(Distance Between The Line and Divot 70b) B}$$
$$= \quad \text{Blade Distal End Position} \qquad \text{(4b)}$$

Here B is a constant coefficient stored in memory 31. Constant coefficient B is identical for all blades.

Determination of blade thickness starts with mathematical generation of position of the longitudinal axis of the blade, step 151. This step is determined by first calculating the midpoint of the straight line between divots 70a and 70c. This point, along with the midpoint of the curve calculated in step 145, are considered to be two points of the longitudinal axis of the blade. Alternatively, the second point is the position of divot 70b. The mathematical definition of this axis points is generated. In a step 152, the distance from divot 70e to the longitudinal axis is determined.

In a step 154, blade thickness is then determined according to the following formula:

$$\text{Blade Thickness} =$$
$$\text{Z (Distance From Long. Axis To Divot 70e) (5)}$$

Here Z is a constant coefficient also stored in processor memory 31. Constant coefficient Z is constant for all blades.

Once blade distal end shape, length, position and thickness are determined, the surgical navigation system of this invention is ready to generate data indicating the position of the blade. These data are then used to produce images of the presence of the blade relative to the surgical site. These data and images are generated using the same methods discussed above with respect to steps 130 and 134 of the first described method of this invention.

An advantage of the above system and method of this invention is that it eliminates the need to store in processor memory 31 a number of different blade definition files. Also this version of the invention eliminates the need to match a reconstructed or generated shape formed on the blade with one of a number of stored shapes.

Figure 10 illustrates a blade 22a with alternative divot pattern from which blade position and profile are determined. Here, blade 22a has a series of divots 158a-g. Each divot 158a-g is disposed in one of 16 locations within a 4x4 grid 160 shown in phantom. In some versions of the invention, divots 158a and 158g are, respectively always located in diagonally opposed locations at corners of the grid 160. Divots 158a and 158g thus serve as reference divots from which the position of the other divots on the grid 160 are determined. Divots 158*b-f* are located in other locations on the grid 160 as a function of blade type.

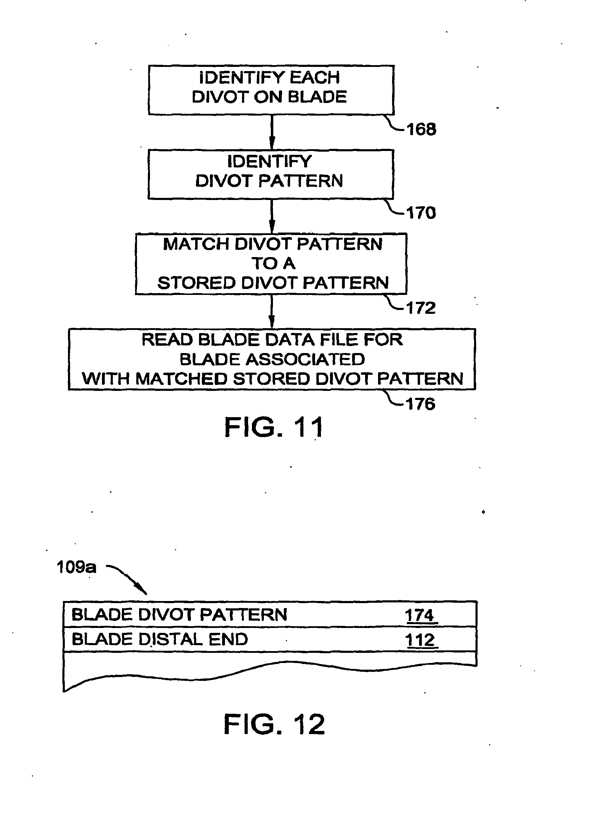

In an equivalent to step 84, step 168 of Figure 11, pointer 88 is used to identify the location of each divot. Then, in step 170, based on the measured position of each divot, the divot pattern is reproduced by the navigation processor 30. In some versions of the invention, this pattern is reproduced based on the position of the divots having "known" locations, divots 158a and 158g. In a step 172, this profile is matched to one of a number of stored profiles in number of blade definition files 109a, one partial shown in Figure 12. Files 109a are substantially identical to the previously described blade definition files 109. However instead of a curve profile data field for the associated blade, the file contains a blade divot pattern field 174. The divot match pattern field contains data describing the divot pattern that is unique to the blade type.

In step 172, a matching process is used to match the divot pattern of the attached blades to one of the stored divot patterns. Processor 30 interprets the match as indicating the blade associated with the matched pattern is attached to the handpiece. Consequently, a step 176, identical to step 120, is executed to read the remainder of the data describing the characteristics of the matched blade into the processor 30. The operation of the saw 24 is controlled as before.

In some versions of this invention, the data in the blade distal end field 112 indicates the distance from the forward most row of grid 160 at which the blade distal end is located.

In still another alternative version of the invention, instead of divots, an elongated groove 177 is formed on the flat of the blade 22a as seen in Figure 10. In this version of the invention, after the blade 22a is mounted to the handpiece 24 and the handpiece head 44 is indexed, in a step similar to step 84 the position and orientation of the groove 177 is obtained. These data are obtained by placing the tip of the pointer 88 in the groove and running the tip along the whole of the length of the groove.

Then, in a step similar to step 96, the shape matching algorithm is used to match the shape of groove 177 to a corresponding shape in one of the blade characteristic files. Thus, as a result of the execution of this step, processor 30 matches the blade to the associated stored blade definition file 109. The previously described steps employed to read the data describing the blade and to operate the saw 24 are executed as before.

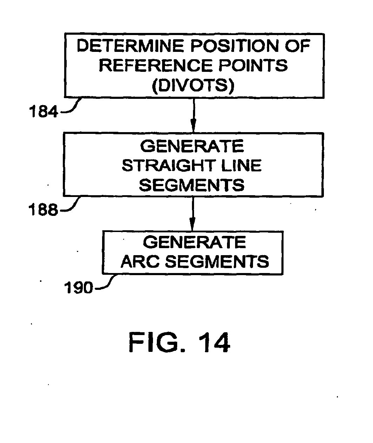

In still another version of the invention, a pattern reconstruction process is employed to determine basic information obtained from a set of divots or other reference points formed on a blade. This method may be employed on a blade such as blade 22b depicted by Figure 13. Here, blade 22b is provided with divots 180a-e. In a step 184, shown in the flow chart of Figure 14, pointer 88 is employed to determine the position of the divots.

Pattern reconstruction starts with generation of the straight line segments of the pattern, step 188. Specifically, processor 130 determines the distance between divots that area spaced apart a distance greater than a distance m. In Figure 15, the two line segments 192 and 194 are shown. In Figure 15, divots 180a-e are represented as points 191a-e.

In a step 190 processor 30 generates an arc between the three divots that are spaced apart a distance less than n. In Figure 15, this curved portion of the pattern is represented by arc 196.

Data defining the characteristics of blade 22b are then obtained based on the reconstructed pattern. In some versions of the invention, the pattern matching algorithm is employed to match the reconstructed pattern to one of the curves, patterns, in a blade definition file 109; a step similar to step 96 is executed.

Alternatively, each segment of the pattern of the pattern is recognized as being proportional to or associated with a specific blade definition characteristic. For example, in some versions of the invention line segment 192 is proportional to (by a known coefficient or constant) the distance from the proximal end of arc 196 to the distal end of the blade 22b. Arc 196 has a unique relationship with a specific distal end blade curvature. Line segment 194 is proportional to the blade thickness. Thus, steps similar to steps 95,96 and 120 are executed to, based on the segments forming the reconstructed pattern, generate data describing the characteristics of the blade 22b.

Alternative versions of the system of this invention are possible. For example, in some versions of the invention, a protective cap may be fitted over the blade. In these versions of the invention, the divots or blade type-defining plots are formed on the cap. In these versions of the invention, it should be clear that the cap is not removed until after the blade is attached to the handpiece and the pointer is used to identify the divots or other blade type-defining plot.

Likewise, it should be clear that the blade type-defining indentations need not always be recessed features formed on the blade flat. In alternative versions of the invention, these features may simply be marking printed or otherwise formed on the blade. In some versions of the invention, these features are raised relative to the flat surfaces of the blade.

In some versions of the invention, these blade defining features may not even be formed or located on the blade flat. For example, in some versions of the invention, the blade-defining features may be notches that extend inwardly from the blade longitudinal side edges. The number of notches, notch lengths and/or inter-notch spacing is unique to each blade type.

When the invention is practiced with the a cutting accessory attached to the described surgical saw, the components internal to the saw typically have such a high cumulative quantity of static friction that, between successive taping off of the pointer 88 to successive divots 70a, 70b and 70c, the saw blade remains static. However, some tools 24 with which this invention may be practiced are not similarly designed. There is a possibility with that between individual divot tap offs, the cutting accessory 22 could move. When the invention is practiced with this type of tool and cutting accessory pair, it may be necessary to first place the tool and/or cutting accessory in jig that holds the cutting accessory static. This assures that since the cutting accessory is immobilized, the divot location determinations made as a result of the successive tap offs will accurately represent the relative positions of the divots.

In some versions of the invention, the data defining the characteristics of the blade may be stored in an RFID or other machine readable memory unit integral with the blade. Thus the RFID or other device is contained in a plastic block. If the memory device is an RFID, the coil through which signals are inductively exchanged with the RFD is also embedded in a block. The block is contained in a window formed in the blade. The saw is formed with a complementary reader.

Alternatively, the RFID or other memory device is attached to the cap fitted over the blade or be contained within the blade packaging.

Therefore, it is an object of the appended claims to cover all such variations and modifications that come within the scope of the claims.

**Claims**

1. A surgical saw blade (22, 22b, 22c) said saw blade including:

    a blade body (23);
    cutting teeth (68) that extend from the blade body (23);
    coupling features (64) integral with the blade body (23) that releaseably engage a surgical saw blade coupling assembly (42) so that the blade body is secured to and actuated by a surgical saw (24);

**characterized in that** the blade body is formed with a plurality of geometric features (70, 158, 180) that are spaced apart from each other, the positions of which are detectable by a surgical navigation pointer (88); wherein relative locations of said plurality of geometric features (70, 158, 180) to each other are a function of at least one selected from the group comprising:

a location of the cutting teeth (68);
a profile of the cutting teeth (68);
a thickness of the blade (22, 22b, 22c);
a width of the blade (22, 22b, 22c);
a length of the blade (22, 22b, 22c); and
a speed at which the blade (22, 22b, 22c) can be actuated.

2. A surgical saw blade (22, 22b, 22c) said saw blade including:

a blade body (23);
cutting teeth (68) that extend from the blade body (23);
coupling features (64) integral with the blade body (23) that releaseably engage a surgical saw blade coupling assembly (42) so that the blade body is secured to and actuated by a surgical saw (24);
**characterized in that** the blade body is formed with at least one geometric feature (70, 158, 170, 180) the position of which is detectable by a surgical navigation pointer (88); wherein
said at least one geometric feature is an elongated slot (170); and
a shape and a size of the elongated slot (170) is a function of at least one selected from the group comprising:

a location of the cutting teeth (68);
a profile of the cutting teeth (68);
a thickness of the blade (22, 22b, 22c);
a width of the blade (22, 22b, 22c);
a length of the blade (22, 22b, 22c); and
a speed at which the blade (22, 22b, 22c) can be actuated.

3. The surgical saw blade of Claim 1 or 2, wherein:

the blade body (23) has a planar surface; and
said at least one geometric feature (70, 158, 170, 180) is a divot or slot formed in the blade body planar surface.

4. A surgical tool system, said system comprising:

a surgical saw (24) having a motor (36) and a coupling assembly (42) for releasably coupling a saw blade (22, 22b, 22c) to the motor (36) so that the saw blade (22, 22b, 22c) will be actuated by said motor (36);
a surgical saw blade (22, 22b, 22c) according to one of claims 1 to 3,
wherein:

the surgical navigation unit (30) includes a pointer (88) for locating the at least one saw blade geometric feature (70, 158, 170, 180);
said surgical navigation unit (30) is configured to determine a shape of a curve (108) defined by the locations of the plurality of geometric features (70, 158, 180), if the at least one geometric feature is the plurality of geometric features, or a shape of the elongated slot (170), if the at least one geometric feature is the elongated slot (170),
said surgical navigation unit (30) is configured to determine, based on the determined shape, at least one from the group of:

the location of the cutting teeth (68);
the profile of the cutting teeth (68);
the thickness of the blade (22, 22b, 22c);
the width of the blade (22, 22b, 22c); and
the length of the blade (22, 22b, 22c); and,

based on the determination made by the surgical navigation unit (30) and the tracked location of the surgical saw (24), the surgical navigation unit generates data indicating the location and orientation of the saw blade (22, 22b, 22c).

5. The surgical tool system of Claim 4 in combination with claim 1 or in combination with claims 1 and 3, wherein:

wherein
the shape is a function of the location of the cutting teeth (68), and
based on the determined shape of said saw blade geometric features (70, 158, 180), the surgical navigation unit (30) is configured to: determine the position of the cutting teeth (68) relative to the surgical saw (24); and based on the position of the cutting teeth (68) and the tracked location of the surgical saw (24), the surgical navigation unit (30) is configured to generate data indicating the location of the saw blade cutting teeth (68).

6. The surgical tool system of Claims 4 or 5, wherein:

the determined shape has shape that is unique to a set of data (109, 109a) that defines the characteristics of the saw blade (22, 22b, 22c); based on the determined shape, the surgical navigation unit match the saw blade (22, 22b, 22c) to the appropriate set of data (96) and read the data (120); and
based on the read data, the surgical navigation unit (30) is configured to determine the position of the saw blade cutting teeth (68).

7. The surgical tool system of Claim 6, wherein the shape is curve (108) or a multi-segment shape that includes straight and arcuate sections (177, 192, 194, 196).

8. The surgical tool system of Claim 6 in combination with at least claim 1, wherein the plurality of saw blade geometric features (70, 158, 180) are a set of divots (70a-c, 158a-g, 180a-e) that have a relationship to a specific set of saw blade data (109, 109a).

9. The surgical tool system of Claims 4, 5, or 6, wherein: the at least one geometric feature (70, 158, 170, 180) is located on the saw blade (22, 22b, 22c) so as to be a defined distance from the saw blade teeth (68); and the surgical navigation unit (30) is configured to determine the position of the saw blade teeth (68) relative to the surgical saw (24) by calculating a position for the saw blade teeth (150) as a function of the location of the saw blade body geometric feature (70, 158, 170, 180).

10. The surgical tool system of Claim 6, wherein:

the set of data (109, 109a) further comprises data (119) indicating a speed at which the saw blade (22, 22b, 22c) should operate; and
the surgical navigation unit (30), based on the speed data (119)generates data indicating the speed at which the saw blade (22, 22b, 22c) should be operated.

11. A method for determining a position of teeth of a surgical saw blade (22, 22b, 22c), the saw blade (22, 22b, 22c) being actuated by a powered handpiece (24), said method including the steps of:

releasably coupling a saw blade (22, 22b, 22c) to a handpiece (24), the saw blade (22, 22b, 22c) having: teeth (68); and a plurality of spaced apart geometric features (70, 158, 180), the relative locations of which are related to a position of the saw blade teeth (68), wherein the positions of the geometric features (70,158,170,180) can be located and the handpiece (24) has a motor (36) for actuating the saw blade (22, 22b, 22c);
with a surgical navigation system (30), tracking the position of the handpiece (24);
the method further comprising the steps of:
with the surgical navigation system (30), determining the relative locations of the plurality of geometric features (70, 158, 170, 180) on the saw blade (22, 22b, 22c);
based on the relative locations of the plurality of saw blade geometric features (70, 158, 170, 180), determining the location of the saw blade teeth (68) relative to the handpiece (24); and
based on the position of the handpiece (24) and the location of the saw blade teeth (68) relative to the handpiece (24), generating data indicating the position of the saw blade teeth (68).

12. The method of determining the position of the teeth of a saw blade of Claim 11, further comprising:

based on the relative locations of the geometric features (70, 158, 180, determining a specific pattern for the geometric features (70, 158, 180); and

in said location or shape matching step, matching the determined pattern of the geometric features (70, 158, 180) to a pattern that is specific to one data set (109, 109a) to determine the matched data set (109, 109a), each data set (109, 109a) including data describing the characteristics of the saw blade (22, 22b, 22c).

13. A method for determining a position of teeth of a surgical saw blade (22, 22b, 22c), the saw blade (22, 22b, 22c) being actuated by a powered handpiece (24), said method including the steps of:

releasably coupling a saw blade (22, 22b, 22c) to a handpiece (24), the saw blade (22, 22b, 22c) having: teeth (68); and a geometric feature (70, 158, 170, 180), the position of which can be located, and the handpiece (24) having a motor (36) for actuating the saw blade (22, 22b, 22c);
with a surgical navigation system (30), tracking the position of the handpiece (24);
Wherein that the geometric feature (70, 158, 170, 180) is an elongated slot (170) having a shape and size that is related to a position of the saw blade teeth (68), the method further comprising the steps of:

with the surgical navigation system (30), determining the size and shape of the elongated slot (170) on the saw blade (22, 22b, 22c);
based on the shape and size of the elongated slot (170), determining the location of the saw blade teeth (68) relative to the handpiece (24); and
based on the position of the handpiece (24) and the location of the saw blade teeth (68) relative to the handpiece (24), generating data indicating the position of the saw blade teeth (68).

14. The method of determining the position of the teeth of a saw blade of Claim 13, wherein said step of determining the location of the saw blade teeth (68) is performed by matching the determined shape of the elongated slot (170) to one from plurality of unique data sets (109, 109a), each data set (109, 109a) including data describing the characteristics of the saw blade (22, 22b, 22c).

15. The method of determining the position of the teeth of a saw blade of Claim 14, further including the steps of:

reading the matched data set (109, 109a); and
based on the data read from the selected data set (109, 109a), determining one of the location of the saw blade teeth (68) and an operating speed for the handpiece motor (36).

**Patentansprüche**

1. Chirurgisches Sägeblatt (22, 22b, 22c), wobei das Sägeblatt enthält:

einen Sägeblattkörper (23);
Schneidzähne (68), die vom Sägeblattkörper (23) ausgehen;
Verbindungseinrichtungen (64) als Bestandteil des Sägeblattkörpers (23), die mit einer Verbindungsanordnung (42) für ein chirurgisches Sägeblatt lösbar verkuppeln, sodass der Sägeblattkörper an einer chirurgischen Säge (24) befestigt ist und von dieser betätigt wird;
**dadurch gekennzeichnet, dass**
der Sägeblattkörper mit mehreren geometrischen Merkmalen (70, 158, 180) ausgeformt ist, die voneinander räumlich beabstandet sind und deren Positionen durch einen chirurgischen Navigationszeiger (88) ermittelt werden können; wobei
relative Positionen der mehreren geometrischen Merkmale (70, 158, 180) zueinander eine Funktion von wenigstens einem aus:

einer Lage der Schneidzähne (68);
einem Profil der Schneidzähne (68);
einer Dicke des Sägeblatts (22, 22b, 22c);
einer Breite des Sägeblatts (22, 22b, 22c);
einer Länge des Sägeblatts (22, 22b, 22c); und
einer Geschwindigkeit, mit der das Sägeblatt (22, 22b, 22c) betrieben werden kann, sind.

**2.** Chirurgisches Sägeblatt (22, 22b, 22c), wobei das Sägeblatt enthält:

einen Sägeblattkörper (23);
Schneidzähne (68), die vom Sägeblattkörper (23) ausgehen;
Verbindungseinrichtungen (64) als Bestandteil des Sägeblattkörpers (23), die mit einer Verbindungsanordnung (42) für ein chirurgisches Sägeblatt lösbar verkuppeln, sodass der Sägeblattkörper an einer chirurgischen Säge (24) befestigt ist und von dieser betätigt wird;
**dadurch gekennzeichnet, dass**
der Sägeblattkörper mit wenigstens einem geometrischen Merkmal (70, 158, 170, 180) ausgeformt ist, dessen Position durch einen chirurgischen Navigationszeiger (88) ermittelt werden kann; wobei
das wenigstens eine geometrische Merkmal ein länglicher Schlitz (170) ist; und eine Form und eine Größe des länglichen Schlitzes (170) eine Funktion von wenigstens einem aus:

einer Lage der Schneidzähne (68);
einem Profil der Schneidzähne (68);
einer Dicke des Sägeblatts (22, 22b, 22c);
einer Breite des Sägeblatts (22, 22b, 22c);
einer Länge des Sägeblatts (22, 22b, 22c); und
einer Geschwindigkeit, mit der das Sägeblatt (22, 22b, 22c) betrieben werden kann, ist.

**3.** Chirurgisches Sägeblatt gemäß Anspruch 1 oder 2, wobei
der Sägeblattkörper (23) eine ebene Oberfläche besitzt; und
das wenigstens eine geometrische Merkmal (70, 158, 170, 180) eine Vertiefung oder ein Schlitz in der ebenen Oberfläche des Sägeblattkörpers ist.

**4.** Chirurgisches Werkzeugsystem, wobei das System umfasst:

eine chirurgische Säge (24) mit einem Motor (36) und einer Verbindungsanordnung (42), um ein Sägeblatt (22, 22b, 22c) lösbar mit dem Motor (36) zu verbinden, sodass das Sägeblatt (22, 22b, 22c) vom Motor (36) angetrieben werden wird;
ein chirurgisches Sägeblatt (22, 22b, 22c) gemäß einem der Ansprüche 1 bis 3, wobei:

die chirurgische Navigationseinheit (30) einen Zeiger (88) enthält, um das wenigstens eine geometrische Merkmal (70, 158, 170, 180) des Sägeblatts zu lokalisieren;
die chirurgische Navigationseinheit (30) dazu ausgebildet ist, eine Form einer Kurve (108) zu bestimmen, die durch die Positionen der mehreren geometrischen Merkmale (70, 158, 180) definiert ist, falls das wenigstens eine geometrische Merkmal eine Mehrzahl geometrischer Merkmale ist, oder eine Form des länglichen Schlitzes (170), falls das wenigstens eine geometrische Merkmal der längliche Schlitz (170) ist,
die chirurgische Navigationseinheit (30) dazu ausgebildet ist, auf Grundlage der bestimmten Form wenigstens eines aus:

der Lage der Schneidzähne (68);
dem Profil der Schneidzähne (68);
der Dicke des Sägeblatts (22, 22b, 22c);
der Breite des Sägeblatts (22, 22b, 22c); und
der Länge des Sägeblatts (22, 22b, 22c) zu bestimmen; und

auf Grundlage der Bestimmung durch die chirurgische Navigationseinheit (30) und der nachverfolgten Lage der chirurgischen Säge (24) die chirurgische Navigationseinheit Daten erzeugt, die die Lage und die Ausrichtung des Sägeblatts (22, 22b, 22c) anzeigen.

**5.** Chirurgisches Werkzeugsystem gemäß Anspruch 4 in Kombination mit Anspruch 1 oder in Kombination mit den Ansprüchen 1 und 3, wobei:

die Form eine Funktion der Position der Schneidzähne (68) ist, und
die chirurgische Navigationseinheit (30) dazu ausgebildet ist, auf Grundlage der bestimmten Form der geometrischen Merkmale des Sägeblatts (70, 158, 180) die Position der Schneidzähne (68) relativ zur chirurgischen Säge (24) zu bestimmen; und die chirurgische Navigationseinheit (30) dazu ausgebildet ist, auf Grundlage der Position der Schneidzähne (68) und der nachverfolgten Lage der chirurgischen Säge (24) Daten zu erzeugen, die die Lage der Schneidzähne (68) des Sägeblatts anzeigen.

6. Chirurgisches Werkzeugsystem gemäß Anspruch 4 oder 5, wobei:

die bestimmte Form eine Form darstellt, die eindeutig einem Datensatz (109, 109a) entspricht, der die Eigenschaften des Sägeblatts (22, 22b, 22c) definiert; die chirurgische Navigationseinheit auf Grundlage der bestimmten Form das Sägeblatt (22, 22b, 22c) mit dem entsprechenden Datensatz abgleicht (96) und die Daten ausliest (120); und

die chirurgische Navigationseinheit (30) dazu ausgebildet ist, auf Grundlage der ausgelesenen Daten die Position der Schneidzähne (68) des Sägeblatts zu bestimmen.

7. Chirurgisches Werkzeugsystem gemäß Anspruch 6, wobei die Form eine Kurve (108) oder eine mehrgliedrige Form ist, die gerade und gekrümmte Abschnitte enthält (177, 192, 194, 196).

8. Chirurgisches Werkzeugsystem gemäß Anspruch 6 in Kombination mit wenigstens Anspruch 1, wobei die mehreren geometrischen Merkmale (70, 158, 180) des Sägeblatts eine Gruppe von Vertiefungen (70a-c, 158a-g, 180a-e) sind, die in Beziehung zu einem spezifischen Satz von Sägeblattdaten (109, 109a) stehen.

9. Chirurgisches Werkzeugsystem gemäß Anspruch 4, 5 oder 6, wobei: das wenigstens eine geometrische Merkmal (70, 158, 170, 180) so auf dem Sägeblatt (22, 22b, 22c) angeordnet ist, dass es sich in einem definierten Abstand von den Sägeblattzähnen (68) befindet; und die chirurgische Navigationseinheit (30) dazu ausgebildet ist, die Position der Sägeblattzähne (68) relativ zur chirurgischen Säge (24) zu bestimmen, indem sie eine Position für die Sägeblattzähne (150) als eine Funktion der Lage der geometrischen Merkmale (70, 158, 170, 180) des Sägeblattkörpers berechnet.

10. Chirurgisches Werkzeugsystem gemäß Anspruch 6, wobei:

der Datensatz (109, 109a) außerdem Daten (119) umfasst, die eine Geschwindigkeit anzeigen, mit der das Sägeblatt (22, 22b, 22c) arbeiten sollte; und

die chirurgische Navigationseinheit (30) auf Grundlage der Geschwindigkeitsdaten (119) Daten erzeugt, die die Geschwindigkeit anzeigen, mit der das Sägeblatt (22, 22b, 22c) betrieben werden sollte.

11. Verfahren zum Bestimmen einer Position von Zähnen eines chirurgischen Sägeblatts (22, 22b, 22c), wobei das Sägeblatt (22, 22b, 22c) durch ein angetriebenes Handstück (24) angetrieben wird und das Verfahren die Schritte enthält:

lösbares Verbinden eines Sägeblatts (22, 22b, 22c) mit einem Handstück (24), wobei das Sägeblatt (22, 22b, 22c) Zähne (68) besitzt sowie mehrere geometrische Merkmale (70, 158, 180), die voneinander räumlich beabstandet sind und deren relative Positionen in Beziehung zu einer Position der Sägeblattzähne (68) stehen, wobei die Positionen der geometrischen Merkmale (70, 158, 170, 180) lokalisiert werden können und das Handstück (24) einen Motor (36) zum Antreiben des Sägeblatts (22, 22b, 22c) besitzt;

Nachverfolgen der Position des Handstücks (24) mithilfe eines chirurgischen Navigationssystems (30); wobei das Verfahren außerdem die Schritte enthält:

Bestimmen der relativen Lagen der mehreren geometrischen Merkmale (70, 158, 170, 180) auf dem Sägeblatt (22, 22b, 22c) mithilfe des chirurgischen Navigationssystems (30);

Bestimmen der Lagen der Sägeblattzähne (68) relativ zum Handstück (24) auf Grundlage der relativen Lagen der mehreren geometrischen Merkmale (70, 158, 170, 180) des Sägeblatts; und

Erzeugen von Daten, die die Position der Sägeblattzähne (68) anzeigen, auf Grundlage der Position des Handstücks (24) und der Lage der Sägeblattzähne (68) relativ zum Handstück (24).

12. Verfahren gemäß Anspruch 11 zum Bestimmen der Position der Zähne eines Sägeblatts, außerdem umfassend:

Bestimmen eines spezifischen Musters für die geometrischen Merkmale (70, 158, 180) auf Grundlage der relativen Lagen der geometrischen Merkmale (70, 158, 180); und

im Verfahrensschritt des Lage- oder Formabgleichs, Abgleichen des bestimmten Musters der geometrischen Merkmale (70, 158, 180) mit einem Muster, das spezifisch für einen einzelnen Datensatz (109, 109a) ist, um den passenden Datensatz zu bestimmen (109, 109a), wobei jeder Datensatz (109, 109a) Daten enthält, die die Eigenschaften des Sägeblatts (22, 22b, 22c) beschreiben.

13. Verfahren zum Bestimmen einer Position von Zähnen eines chirurgischen Sägeblatts (22, 22b, 22c), wobei das Sägeblatt (22, 22b, 22c) durch ein angetriebenes Handstück (24) angetrieben wird, und das Verfahren die Schritte enthält:

lösbares Verbinden eines Sägeblatts (22, 22b, 22c) mit einem Handstück (24), wobei das Sägeblatt (22, 22b, 22c) Zähne (68) besitzt sowie ein geometrisches Merkmal (70, 158, 170, 180), dessen Position lokalisiert werden kann, und das Handstück (24) einen Motor (36) zum Betreiben des Sägeblatts (22, 22b, 22c) besitzt; Nachverfolgen der Position des Handstücks (24) mithilfe eines chirurgischen Navigationssystems (30); wobei das geometrische Merkmal (70, 158, 170, 180) ein länglicher Schlitz (170) ist, dessen Form und Größe in Beziehung zu einer Position der Sägeblattzähne (68) stehen; wobei das Verfahren außerdem die Schritte umfasst:

Bestimmen der Größe und Form des länglichen Schlitzes (170) auf dem Sägeblatt (22, 22b, 22c) mithilfe des chirurgischen Navigationssystems (30); Bestimmen der Lage der Sägeblattzähne (68) relativ zum Handstück (24) auf Grundlage der Form und Größe des länglichen Schlitzes (170); und Erzeugen von Daten, die die Position der Sägeblattzähne (68) anzeigen, auf Grundlage der Position des Handstücks (24) und der Lage der Sägeblattzähne (68) relativ zum Handstück (24).

14. Verfahren gemäß Anspruch 13 zum Bestimmen der Position der Zähne eines Sägeblatts, wobei der Verfahrensschritt der Lagebestimmung der Sägeblattzähne (68) durch Abgleichen der ermittelten Form des länglichen Schlitzes (170) mit einem von mehreren eindeutigen Datensätzen (109, 109a) geschieht, wobei jeder Datensatz (109, 109a) Daten enthält, die die Eigenschaften des Sägeblatts (22, 22b, 22c) beschreiben.

15. Verfahren gemäß Anspruch 14 zum Bestimmen der Position der Zähne eines Sägeblatts, das außerdem die folgenden Schritte enthält:

Auslesen des passenden Datensatzes (109, 109a); und auf Grundlage der aus dem ausgewählten Datensatz (109, 109a) ausgelesenen Daten, Bestimmen der Lage der Sägeblattzähne (68) oder einer Betriebsgeschwindigkeit für den Handstückmotor (36).

**Revendications**

1. Lame (22, 22b, 22c) de scie chirurgicale, ladite lame de scie incluant :

un corps (23) de lame ; des dents coupantes (68) qui s'étendent à partir du corps (23) de lame ; des caractéristiques de couplage (64) intégrées au corps (23) de lame qui engagent de manière libérable un ensemble de couplage (42) de lame de scie chirurgicale de telle sorte que le corps de lame est fixé à et actionné par une scie chirurgicale (24) ; **caractérisée en ce que** le corps de lame est formé avec une pluralité de caractéristiques géométriques (70, 158, 180) qui sont espacées les unes des autres, les positions desquelles sont détectables par un pointeur (88) de navigation chirurgicale ; dans laquelle des emplacements relatifs de ladite pluralité de caractéristiques géométriques (70, 158, 180) les unes par rapport aux autres sont une fonction d'au moins un choisi parmi le groupe comprenant :

un emplacement des dents coupantes (68) ; un profil des dents coupantes (68) ; une épaisseur de la lame (22, 22b, 22c) ; une largeur de la lame (22, 22b, 22c) ; une longueur de la lame (22, 22b, 22c) ; et une vitesse à laquelle la lame (22, 22b, 22c) peut être actionnée.

2. Lame (22, 22b, 22c) de scie chirurgicale, ladite lame de scie incluant :

un corps (23) de lame ;

des dents coupantes (68) qui s'étendent à partir du corps (23) de lame ;

des caractéristiques de couplage (64) intégrées au corps (23) de lame qui engagent de manière libérable un ensemble de couplage (42) de lame de scie chirurgicale de telle sorte que le corps de lame est fixé à et actionné par une scie chirurgicale (24) ;

**caractérisée en ce que** le corps de lame est formé avec au moins une caractéristique géométrique (70, 158, 170, 180), la position de laquelle est détectable par un pointeur (88) de navigation chirurgicale ; dans laquelle ladite au moins une caractéristique géométrique est une fente allongée (170) ; et

une forme et une taille de la fente allongée (170) sont une fonction d'au moins un choisi parmi le groupe comprenant :

un emplacement des dents coupantes (68) ;

un profil des dents coupantes (68) ;

une épaisseur de la lame (22, 22b, 22c) ;

une largeur de la lame (22, 22b, 22c) ;

une longueur de la lame (22, 22b, 22c) ; et

une vitesse à laquelle la lame (22, 22b, 22c) peut être actionnée.

3. Lame de scie chirurgicale selon la revendication 1 ou 2, dans laquelle :

le corps (23) de lame a une surface plane ; et

ladite au moins une caractéristique géométrique (70, 158, 170, 180) est un divot ou une fente formé(e) dans la surface plane de corps de lame.

4. Système d'outil chirurgical, ledit système comprenant :

une scie chirurgicale (24) ayant un moteur (36) et un ensemble de couplage (42) pour coupler de manière libérable une lame (22, 22b, 22c) de scie au moteur (36) de façon à ce que la lame (22, 22b, 22c) de scie soit actionnée par ledit moteur (36) ;

une lame (22, 22b, 22c) de scie chirurgicale selon l'une des revendications 1 à 3,

dans lequel :

l'unité de navigation chirurgicale (30) inclut un pointeur (88) pour localiser l'au moins une caractéristique géométrique (70, 158, 170, 180) de lame de scie ;

ladite unité de navigation chirurgicale (30) est configurée pour déterminer une forme d'une courbe (108) définie par les emplacements de la pluralité de caractéristiques géométriques (70, 158, 180), si l'au moins une caractéristique géométrique est la pluralité de caractéristiques géométriques, ou une forme de la fente allongée (170), si l'au moins une caractéristique géométrique est la fente allongée (170),

ladite unité de navigation chirurgicale (30) est configurée pour déterminer, sur la base de la forme déterminée, au moins un parmi le groupe de :

l'emplacement des dents coupantes (68) ;

le profil des dents coupantes (68) ;

l'épaisseur de la lame (22, 22b, 22c) ;

la largeur de la lame (22, 22b, 22c) ;

la longueur de la lame (22, 22b, 22c) ; et,

sur la base de la détermination réalisée par l'unité de navigation chirurgicale (30) et de l'emplacement suivi de la scie chirurgicale (24), l'unité de navigation chirurgicale génère des données indiquant l'emplacement et l'orientation de la lame (22, 22b, 22c) de scie.

5. Système d'outil chirurgical selon la revendication 4 en combinaison avec la revendication 1 ou en combinaison avec les revendications 1 et 3, dans lequel :

la forme est une fonction de l'emplacement des dents coupantes (68), et

sur la base de la forme déterminée desdites caractéristiques géométriques (70, 158, 180) de lame de scie, l'unité de navigation chirurgicale (30) est configurée pour : déterminer la position des dents coupantes (68) par rapport à la scie chirurgicale (24) ; et sur la base de la position des dents coupantes (68) et de l'emplacement suivi de la scie chirurgicale (24), l'unité de navigation chirurgicale (30) est configurée pour générer des données indiquant l'emplacement des dents coupantes (68) de lame de scie.

6. Système d'outil chirurgical selon la revendication 4 ou 5, dans lequel :

la forme déterminée a une forme qui est unique pour un ensemble de données (109, 109a) qui définissent les caractéristiques de la lame (22, 22b, 22c) de scie ;
sur la base de la forme déterminée, l'unité de navigation chirurgicale met en correspondance la lame (22, 22b, 22c) de scie avec l'ensemble de données (96) approprié et lit les données (120) ; et
sur la base des données lues, l'unité de navigation chirurgicale (30) est configurée pour déterminer la position des dents coupantes (68) de lame de scie.

7. Système d'outil chirurgical selon la revendication 6, dans lequel la forme est une courbe (108) ou une forme à segments multiples qui inclut des sections rectilignes et en arc (177, 192, 194, 196).

8. Système d'outil chirurgical selon la revendication 6 en combinaison avec au moins la revendication 1, dans lequel la pluralité de caractéristiques géométriques (70, 158, 180) de lame de scie sont un ensemble de divots (70a-c, 158a-g, 180a-e) qui ont une relation avec un ensemble spécifique de données (109, 109a) de lame de scie.

9. Système d'outil chirurgical selon la revendication 4, 5 ou 6, dans lequel : l'au moins une caractéristique géométrique (70, 158, 170, 180) est située sur la lame (22, 22b, 22c) de scie de manière à être à une distance définie des dents (68) de lame de scie ; et l'unité de navigation chirurgicale (30) est configurée pour déterminer la position des dents (68) de lame de scie par rapport à la scie chirurgicale (24) en calculant une position des dents (150) de lame de scie comme une fonction de l'emplacement de la caractéristique géométrique (70, 158, 170, 180) de corps de lame de scie.

10. Système d'outil chirurgical selon la revendication 6, dans lequel :

l'ensemble de données (109, 109a) comprend en outre des données (119) indiquant une vitesse à laquelle la lame (22, 22b, 22c) de scie doit opérer ; et
l'unité de navigation chirurgicale (30), sur la base des données (119) de vitesse; génère des données indiquant la vitesse à laquelle la lame (22, 22b, 22c) de scie doit être opérée.

11. Procédé pour déterminer une position de dents d'une lame (22, 22b, 22c) de scie chirurgicale, la lame (22, 22b, 22c) de scie étant actionnée par une pièce à main (24) motorisée, ledit procédé incluant les étapes de :

couplage libérable d'une lame (22, 22b, 22c) de scie à une pièce à main (24), la lame (22, 22b, 22c) de scie ayant : des dents (68) ; et une pluralité de caractéristiques géométriques (70, 158, 180) espacées, les emplacements relatifs desquelles sont liés à une position des dents (68) de lame de scie, dans lequel les positions des caractéristiques géométriques (70, 158, 170, 180) peuvent être localisées et la pièce à main (24) a un moteur (36) pour actionner la lame (22, 22b, 22c) de scie ;
avec un système de navigation chirurgicale (30), suivi de la position de la pièce à main (24) ;
le procédé comprenant en outre les étapes de :

avec le système de navigation chirurgicale (30), détermination des emplacements relatifs de la pluralité de caractéristiques géométriques (70, 158, 170, 180) sur la lame (22, 22b, 22c) de scie ;
sur la base des emplacements relatifs de la pluralité de caractéristiques géométriques (70, 158, 170, 180) de lame de scie, détermination de l'emplacement des dents (68) de lame de scie par rapport à la pièce à main (24) ; et
sur la base de la position de la pièce à main (24) et de l'emplacement des dents (68) de lame de scie par rapport à la pièce à main (24), génération de données indiquant la position des dents (68) de lame de scie.

12. Procédé de détermination de la position des dents d'une lame de scie selon la revendication 11, comprenant en outre :

sur la base des emplacements relatifs des caractéristiques géométriques (70, 158, 180), la détermination d'un motif spécifique pour les caractéristiques géométriques (70, 158, 180) ; et
à ladite étape de localisation ou de mise en correspondance de formes, la mise en correspondance du motif déterminé des caractéristiques géométriques (70, 158, 180) avec un motif qui est spécifique à un ensemble de données (109, 109a) pour déterminer l'ensemble de données (109, 109a) mis en correspondance, chaque ensemble de données (109, 109a) incluant des données décrivant les caractéristiques de la lame (22, 22b, 22c) de scie.

13. Procédé de détermination d'une position de dents d'une lame (22, 22b, 22c) de scie chirurgicale, la lame (22, 22b, 22c) de scie étant actionnée par une pièce à main (24) motorisée, ledit procédé incluant les étapes de :

couplage libérable d'une lame (22, 22b, 22c) de scie à une pièce à main (24), la lame (22, 22b, 22c) de scie ayant : des dents (68) ; et une caractéristique géométrique (70, 158, 170, 180), la position de laquelle peut être localisée, et la pièce à main (24) ayant un moteur (36) pour actionner la lame (22, 22b, 22c) de scie ; avec un système de navigation chirurgicale (30), suivi de la position de la pièce à main (24) ; dans lequel la caractéristique géométrique (70, 158, 170, 180) est une fente allongée (170) ayant une forme et une taille qui se rapportent à une position des dents (68) de lame de scie, le procédé comprenant en outre les étapes de :

avec le système de navigation chirurgicale (30), détermination de la taille et de la forme de la fente allongée (170) sur la lame (22, 22b, 22c) de scie ; sur la base de la taille et de la forme de la fente allongée (170), détermination de l'emplacement des dents (68) de lame de scie par rapport à la pièce à main (24) ; et sur la base de la position de la pièce à main (24) et de l'emplacement des dents (68) de lame de scie par rapport à la pièce à main (24), génération de données indiquant la position des dents (68) de lame de scie.

14. Procédé de détermination de la position des dents d'une lame de scie selon la revendication 13, dans lequel ladite étape de détermination de l'emplacement des dents (68) de lame de scie est mise en oeuvre en mettant en correspondance la forme déterminée de la fente allongée (170) avec un parmi une pluralité d'ensembles de données (109, 109a) uniques, chaque ensemble de données (109, 109a) incluant des données décrivant les caractéristiques de la lame (22, 22b, 22c) de scie.

15. Procédé de détermination de la position des dents d'une lame de scie selon la revendication 14, incluant en outre les étapes de :

lecture des ensembles de données (109, 109a) mis en correspondance ; et sur la base des données lues dans les ensembles de données (109, 109a) sélectionnés, détermination d'un parmi l'emplacement des dents (68) de lame de scie et une vitesse opérationnelle pour le moteur (36) de pièce à main.

FIG. 1

FIG. 2

EP 1 945 125 B1

FIG. 3

FIG. 10

FIG. 13

EP 1 945 125 B1

```
            ┌─────────────────────────┐
            │   ATTACH SAW BLADE      │
            │    TO HANDPIECE         │
            └─────────────────────────┘
  76 ⌐                    │
            ┌─────────────────────────┐
            │   INDEX HANDPIECE       │
            │   HEAD/SAW BLADE        │
            └─────────────────────────┘
  80 ⌐                    │
            ┌─────────────────────────┐
            │  SET NAVIGATION SYS.    │
            │  TO TRACK HANDPIECE     │
            └─────────────────────────┘
  82 ⌐                    │
            ┌─────────────────────────┐
            │    LOCATE BLADE         │
            │    DISTAL END           │
            │  (DISTAL END DIVOTS)    │
            └─────────────────────────┘
  84 ⌐                    │
            ┌─────────────────────────┐
            │  DEFINE BLADE CURVE     │
            │    FROM DIVOTS          │
            └─────────────────────────┘
  95 ⌐                    │
            ┌─────────────────────────┐
            │  MATCH BLADE CURVE      │
            │   TO STORED CURVE       │
            └─────────────────────────┘
  96 ⌐                    │
      ┌───────────────────────────────┐
      │   READ BLADE DATA FILE FOR    │
      │      BLADE ASSOCIATED         │
      │ WITH MATCHED STORED CURVE     │
      └───────────────────────────────┘
 120 ⌐                    │
                         (A)
```

# FIG. 4A

Ⓐ

```
DETERMINE BLADE DISTAL END
POSITION, ORIENTATION, PROFILE
& TOOTH GEOMETRY
```
122

124

```
BLADE SPEED
=
HP DEFAULT SPEED
?
```
Y

N

```
RESET HP SPEED
TO BLADE SPEED
```
126

```
GENERATE BLADE DISTAL END
POSITION DATA
```
130

```
TRACK SAW BLADE
TEETH
```
132

```
GENERATE IMAGE OF
BLADE DISTAL END
AT SURGICAL SITE
```
134

## FIG. 4B

FIG. 5

191e
194
191d
196
191c
191a
192
191b

FIG. 15

104
108
106
123
102
22
38

FIG. 6

| CURVE DEFINITION | 110 |
| DIVOT COUNT | 111 |
| BLADE DISTAL END | 112 |
| SHAPE DATA | 114 |
| OPERATING CHARACTERISTICS | 116 |

109

## FIG. 7

DETERMINE BLADE WIDTH — 146

↓

DETERMINE BLADE
DISTAL END GEOMETRY — 148

↓

DETERMINE POSITION (LOCATION)
OF BLADE DISTAL END — 150

↓

DETERMINE BLADE
LONGITUDINAL AXIS — 151

↓

DETERMINE DISTANCE:
REFERENCE POINT (DIVOT)
TO LONGITUDINAL AXIS — 152

↓

DETERMINE
BLADE THICKNESS — 154

## FIG. 9

140

22

142

138

FIG. 8

```
┌─────────────────────────┐
│     IDENTIFY EACH       │
│    DIVOT ON BLADE       │
└─────────────────────────┘
            │           └─168
            ▼
┌─────────────────────────┐
│       IDENTIFY          │
│    DIVOT PATTERN        │
└─────────────────────────┘
            │           └─170
            ▼
┌─────────────────────────┐
│  MATCH DIVOT PATTERN    │
│        TO A             │
│  STORED DIVOT PATTERN   │
└─────────────────────────┘
            │           └─172
            ▼
┌─────────────────────────────────┐
│   READ BLADE DATA FILE FOR      │
│      BLADE ASSOCIATED           │
│ WITH MATCHED STORED DIVOT PATTERN│
└─────────────────────────────────┘
                       └─176
```

## FIG. 11

109a

| BLADE DIVOT PATTERN | 174 |
|---------------------|-----|
| BLADE DISTAL END | 112 |

## FIG. 12

DETERMINE POSITION OF
REFERENCE POINTS
(DIVOTS)

184

GENERATE
STRAIGHT LINE
SEGMENTS

188

GENERATE
ARC SEGMENTS

190

# FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 10239710
- US 20050154296 A
- US 67787403 A
- US 20040073279 A1
- US 69931505 P
- US 69459205 P